# EUROPÄISCHE PATENTSCHRIFT

(11) **EP 0 355 679 B1**
(45) Veröffentlichungstag und Bekanntmachung des Hinweises auf die Patenterteilung: **28.02.1996**
(21) Anmeldenummer: 89115069.0
(22) Anmeldetag: 16.08.1989
(51) Int. Cl.: C07H 13/12, C07C 59/60, C07F 9/09, C12N 1/20, C12N 9/16, C12P 19/26, C12P 7/42

(54) **Neuer Mikroorganismus zum Abbau von Moenomycinen, Verfahren zum Abbau sowie die Verwendung der Abbauprodukte**
Microorganism for the degradation of moenomycins, process for the degradation and use of the degradation products
Microorganisme pour la dégradation de moénomycines, procédé de dégradation et utilisation des produits de la dégradation

(30) Priorität: 20.08.1988 DE 3828337
(43) Veröffentlichungstag der Anmeldung: 28.02.1990
(73) Patentinhaber: HOECHST AKTIENGESELLSCHAFT, D-65926 Frankfurt am Main (DE)
(72) Erfinder: Aretz, Werner, Dr., D-6240 Königstein/Taunus (DE); Böttger, Dirk, Dr., D-6274 Hünstetten 3 (DE); Seibert, Gerhard, Prof. Dr., D-6100 Darmstadt (DE); Tumulka, Alois, Dr., D-6240 Königstein/Taunus (DE); Welzel, Peter, Prof. Dr., D-4630 Bochum (DE); Hobert, Kurt, D-4630 Bochum (DE)

(56) Entgegenhaltungen:
- EP-A- 0 130 327
- GB-A- 1 111 024

## Beschreibung

Moenomycin A (Abb. 1) ist die Hauptkomponente des in der Tierernährung verwendeten Flavomycin®. Wie andere bekannte Phosphoglycolipidantibiotika hemmt es die Biosynthese des Peptidoglycangerüsts der bakteriellen Zellwand. Genauere Untersuchungen zeigten, daß die Transglycosylierungsreaktion des Penicillin-Bindeproteins 1b von E. coli durch diese Stoffe inhibiert wird. [Huber G., Antibiotics, V-1, Seiten 135 bis 153, (1979)]. Versuche, Phosphoglykolipidantibiotika enzymatisch bzw. mikrobiell gezielt abzubauen, scheiterten bislang.

Aus der EP-A-0 130 327 ist ein Verfahren bekannt, mit dem allerdings erst nach Hydrierung der ungesättigten Alkylgruppe des Moenomycins A (bzw. des Moenomycin-A-Bestandteils I) die Moenomycin-A-Bestandteile A, B, C, D und E, welche zum Oligosaccharidrest des Moenomycins gehören, der aus den Bestandteilen A, B, C, D, E und F besteht, auf chemischen Weg sukzessiv abgespalten werden können.

Überraschend wurde nun aus einem kontaminierten Fermenter zur Herstellung von Flavomycin mit Streptomyces ghanaensis eine neue Bacillus spezies isoliert, die die Phosphoglykolipidantibiotika zu definierten Endprodukten spalten kann. Diese Endprodukte besitzen antibiotische Aktivität oder können als Synthesebausteine für neue Transglycosylase Inhibitoren verwendet werden.

Die Erfindung betrifft somit:
1. Bacillus spec. DSM 4675 sowie seine Varianten und Mutanten.
2. Das Spaltprodukt des Moenomycin A mit der Formel I 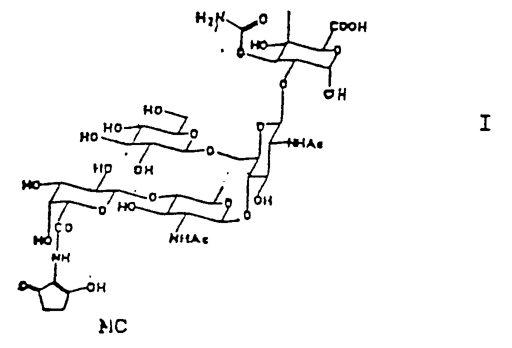
3. Das Spaltprodukt der Phosphoglykolipidantibiotika mit der allgemeinen Formel, 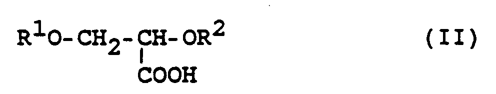 in der R¹ Wasserstoff oder eine Phosphonogruppe [-PO(OH)₂] und R² eine (C₅ bis C₅₅)-Alkylgruppe ist, die verzweigt oder unverzweigt, gesättigt oder ungesättigt sein kann.
4. Die Enzyme mit deren Hilfe die Phosphoglykolipidantibiotika an der phosphoglykosidischen Bindung bzw. das unter 3. charakterisierte Spaltprodukt an der Monophosphatester Bindung gespalten werden können.
5. Ein Verfahren zur Herstellung der unter 2. und 3. charakterisierten Abbauprodukte, das dadurch gekennzeichnet ist, daß Phosphoglykolipidantibiotika mit Bacillus spec. DSM 4675 inkubiert werden.
6. Die Verwendung der unter 2. und 3. charakterisierten Substanzen als Synthesebaustein zur Herstellung von Transglycosylase Inhibitoren bzw. als antibiotisch wirksamer Stoff.

Die Erfindung wird im folgenden detailliert beschrieben, insbesondere in den bevorzugten Ausführungsformen. Ferner wird sie in den Ansprüchen definiert.

Bacillus spec. wurde mit der Nummer DSM 4675 unter den Bedingungen des Budapester Vertrags bei der Deutschen Sammlung von Mikroorganismen und Zellkulturen GmbH in Braunschweig, BRD, am 23.06.1988 hinterlegt. Der Stamm kann wie folgt charakterisiert werden:

### 1. Taxonomische Eigenschaften von Bacillus sp. DSM 4675

**A) Morphologie**
- bewegliche Stäbchen; bis 5 µm lang; teilweise in kurzen Ketten
- endständige Spore; Sporangium geschwollen
- Gram-positiv.

**B) Wachstum auf verschiedenen Medien (28°C; 48 Stunden)**
1. Antibiotic-Medium 3, (Difco)
   - rauhe, gelappte Kolonien von 1 - 2 mm Durchmesser
2. Luria broth (Bacto-Trypton 10 g/l; Bacto-Yeast 5 g/l; NaCl 5 g/l)
   - glatte, glänzende, runde Kolonien von 2 - 3 mm Durchmesser; opak
3. Nutrient broth, (Difco)
   - weiße, glänzende Kolonien mit unregelmäßigem Rand
4. Harnstoffagar nach Christensen, (Difco)
   - Wachstum positiv
5. Mc Conkey Agar, (Difco)
   - Wachstum positiv
6. BROLAC-Agar (Lactose), (Difco)
   - Wachstum positiv
7. Simmons-Citrat-Agar, (Difco)
   - Wachstum negativ
8. kein Wachstum in Gegenwart von 7 bzw. 10 % NaCl in einem Pepton-Fleischextrakt Medium, (Difco)

**C) Physiologische Eigenschaften**

| | | | |
|---|---|---|---|
| 1. | Oxidase | | + |
| 2. | Katalase | | + |
| 3. | Hämolyse | | - |
| 4. | Aminopeptidase | | - |
| 5. | Nitratreduktion | | - |
| 6. | Phenylalanin-Desaminase | | - |
| 7. | Wachstum bei | 30°C | + |
| | | 40°C | + |
| | | 50°C | + |
| 8. | anaerobes Wachstum | | |
| | | - fest | - |
| | | - flüssig | - |
| 9. | Gasbildung aus Glucose | | - |
| 10. | Indolbildung | | - |
| 11. | Arginindihydrase | | - |
| 12. | Harnstoffabbau | | - |
| 13. | Eskulin Hydrolyse | | + |
| 14. | Gelatineabbau | | - |
| 15. | β-Galactosidase | | + |
| 16. | Lysindecarboxylase | | - |
| 17. | Ornithindecarboxylase | | - |
| 18. | H₂S-Produktion | | - |
| 19. | Tryptophan-Desaminase | | - |
| 20. | alkal. Phosphatase | | - |
| 21. | Voges-Proskauer-Reaktion | | - |

**D) Fermentation von Kohlenhydraten**

| C-Quelle | Assimilation | Säurebildung |
|---|---|---|
| Adipat | - | |
| Adonit | - | |
| Arabinose | + | + |
| Caprat | - | |
| Citrat | - | |
| Dulcit | - | |
| Fructose | + | + |
| Galactose | - | |
| Gluconat | + | |
| Glucose | + | + |
| Inosit | - | |
| Lactose | + | + |
| Malat | - | |
| Malonat | - | |
| Maltose | + | + |
| Mannit | + | + |
| Mannose | + | + |
| Melibiose | + | + |
| Phenylacetat | - | |
| Raffinose | + | + |
| Rhamnose | - | |
| Saccharose | + | + |
| Salicin | - | |
| Sorbit | - | |
| Trehalose | + | + |
| Xylit | - | - |
| Xylose | + | + |
| N-Acetyl-Glucosamin | - | + |

Unter Berücksichtigung taxonomischer Merkmale und Zuhilfenahme von "Bergey's Manual of Systematic Bacteriology" (Vol. 2, Williams and Wilkins publ., Baltimore, 1986) ist der Stamm der Gattung Bacillus zuzuordnen. Zur Bestimmung der Art wurden vergleichsweise Typkulturen von Bacillus macerans, circulans, lentus, alcalophilus, stearothermophilus, licheniformis, polymyxa und fastidiosus parallel überprüft. Alle Vergleichsstämme zeigten deutliche Unterschiede zu Bacillus spec. DSM 4675 auf. Es konnte auch keiner dieser Stämme Phosphoglykolipidantibiotika, insbesondere Moenomycin-A, abbauen. Daraus ist zu schließen, daß der Stamm DSM 4675 eine neue Art ist.

Die Erfindung betrifft auch jeweils die Mutanten und Varianten, die wie bekannt spontan entstehen können bzw. durch Behandlung mit physikalischen Mitteln, beispielsweise Bestrahlung, wie Ultraviolett- oder Röntgenstrahlen oder mit chemischen Mutagenen, wie beispielsweise Ethylmethansulfonat (EMS), N-Methyl-N′-nitro-N-nitroso-guanidin (MNNG) oder 2-Hydroxy-4-methoxy-benzophenon (MOB) erzeugt werden.

Als bevorzugte Kohlenstoffquelle für die aerobe Fermentation von Bacillus spec. DSM 4675 eignen sich assimilierbare Kohlenhydrate und Zuckeralkohole, wie Glucose, Lactose oder Mannit, sowie kohlenhydrathaltige Naturprodukte, wie Malzextrakt. Als bevorzugte stickstoffhaltige Nährstoffe kommen in Betracht: Aminosäuren, Peptide und Proteine sowie deren Abbauprodukte, wie Peptone oder Tryptone, ferner Fleischextrakte, gemahlene Samen, beispielsweise von Mais, Bohnen, Soja oder der Baumwollpflanze, Destillationsrückstände der Alkoholherstellung, Fleischmehle oder Hefeextrakte, aber auch Ammoniumsalze und Nitrate. Außerdem kann die Nährlösung beispielsweise Chloride, Carbonate, Sulfate oder Phosphate der Alkali-oder Erdalkalimetalle, Eisen, Zink und Mangan als zusätzliche anorganische Salze enthalten.

Das Wachstum des Mikroorganismus und die Bildung der für die erfindungsgemäßen Abbaureaktionen notwendigen Enzyme ist besonders gut in einem Nährmedium mit Maisstärke, Sojamehl, Saccharose, Glycerin, Pepton und/oder Cornsteep als Kohlenstoff- bzw. Stickstoffquelle.

Die Fermentation erfolgt aerob, also beispielsweise submers unter Schütteln oder Rühren in Schüttelkolben oder Fermentern, gegebenenfalls unter Einführen von Luft oder Sauerstoff. Die Fermentation kann in einem Temperaturbereich von etwa Raumtemperatur bis 50°C stattfinden, vorzugsweise bei etwa 35 bis 37°C. Die Kulturzeit beträgt im allgemeinen 24 bis 48 Stunden.

Die so erhaltenen Bacilluskulturen von DSM 4675 oder Präparationen davon können zur Spaltung der Phosphoglykolipidantibiotika eingesetzt werden. Dazu gehören insbesondere die Antibiotika der Moenomycingruppe, beispielsweise das Pholipomycin¹⁾, die Prasinomycine²⁾, die Diumycine (Macarbomycine)³⁾ Esanchomycin, Prenomycin und Teichomycin, sowie andere strukturell verwandte Substanzen, die eine entsprechend funktionalisierte Phosphoglycerinsäure aufweisen
[1) S. Takahashi et al., Tetrahedron Lett. 1983, 499
2) F.L. Weisenborn et al., Nature 213, 1092 (1967)
3) S. Takahashi et al., J. Antibiot. 26, 542 (1973)].

Insbesondere bevorzugt werden mit den Enzymen die Moenomycine, beispielsweise das Flavomycin, abgebaut.

Wenn Bacillus-Zellen verwendet werden, ist es vorteilhaft, diese zu permeabilisieren, beispielsweise mit Cetyltrimethylammoniumsalzen. Es ist ebenfalls möglich, mit Proteinisolaten aus den Bacillus-Zellen, bzw. mit beispielsweise durch Aussalzen oder Chromatographie partiell angereicherten Enzym-Extrakten oder natürlich mit dem gereinigten Enzym zu arbeiten. Ferner ist es möglich Enzym und Zellen in freier oder immobilisierter Form einzusetzen.

Der enzymatische Abbau ist am Beispiel von Moenomycin A im folgenden Schema dargestellt.

Enzymatischer Abbau von Moenomycin A 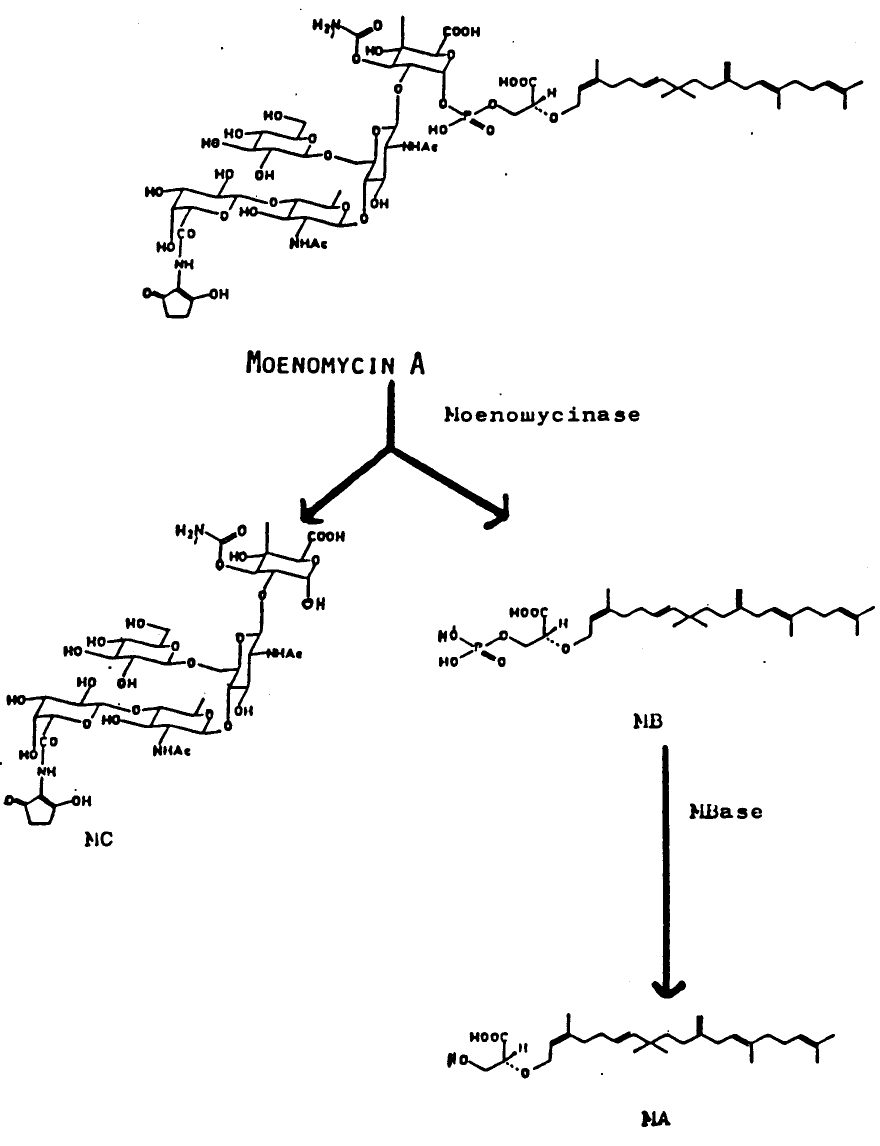
Aus diesem Schema geht hervor, daß zur Herstellung der Spaltprodukte zwei Enzyme notwendig sind. Zur Spaltung der phosphoglykosidischen Bindung wird ein Enzym benötigt, das von den Erfindern Moenomycinase genannt wurde. Moenomycinase ist mit der Cytoplasmamembran von Bacillus spec. DSM 4675 assoziiert und kann nach an sich bekannten Methoden zur Enzymisolierung aus dem Mikroorganismus gewonnen werden.

Die Moenomycinase hat ein pH-Optimum von ca. 8,0-8,5, insbesondere 8,2-8,3. Das Temperatur-Optimum des Enzyms liegt bei 45-55°C, insbesondere bei 49-51°C. Die Moenomycinase hat einen Kₘ-Wert für Moenomycin A von 4-10 mmolar.

Man erhält zwei Spaltprodukte. Das Spaltprodukt I besteht aus der Zuckerkomponente der Phosphoglykolipidantibiotika. Ferner erhält man das Spaltprodukt mit der allgemeinen Formel II, 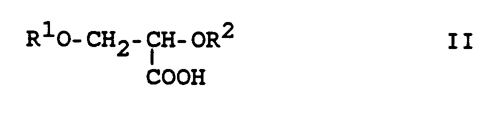
in der R¹ eine Phosphonogruppe und R² eine (C₅ bis C₅₅)-Alkylgruppe, bevorzugt eine (C₁₀ bis C₃₀)-Alkylgruppe, insbesondere eine (C₂₀ bis C₂₅)-Alkylgruppe ist, die jeweils verzweigt oder unverzweigt, gesättigt oder ungesättigt, vorzugsweise verzweigt und ungesättigt sein können.

Vorzugsweise werden Moenomycine als Substrate eingesetzt, so daß man als Spaltprodukte die der Verbindung MC entsprechenden Stoffe sowie die Verbindungen MB und MA erhält (siehe Formelschema).

Zur Dephosphorylierung des durch Inkubation mit Moenomycinase erhaltenen Phosphoglycerinsäurelipids der allgemeinen Formel II in der R¹ Wasserstoff ist, wird ein weiteres Enzym benötigt, das ebenfalls aus dem erfindungsgemäßen Mikroorganismus gewonnen werden kann. Dieses Enzym wurde von den Erfindern MBase genannt. MBase kann ebenfalls nach an sich bekannten Methoden aus dem Mikroorganismus isoliert werden. Beispielsweise werden die Zellen mit Ultraschall aufgeschlossen und der resultierende Rohextrakt entweder durch Ammoniumsulfatfraktionierung (25-55 % Sättigung) oder Ultrazentrifugation weiter angereichert. Danach erfolgt Dialyse. Abschließend werden die Moenomycinase und MBase chromatographisch getrennt.

Die MBase wird bei Temperaturen, die über 37°C liegen, sowie im sauren pH-Bereich ab pH 5 bei sinkendem pH zunehmend inaktiviert.

Die Spaltung der Moenomycine sowie der Phosphoglykolipidantibiotika kann, wie schon erwähnt, mit ganzen Zellen oder Enzymisolaten durchgeführt werden.

Die Reaktion erfolgt im allgemeinen in wäßrigem Medium, bei einem pH-Wert von ca. 5,5-8,5, bevorzugt pH 7-8. Die Umsetzungszeit beträgt im allgemeinen 5-48 Stunden bevorzugt ca. 24 Stunden. Die Reaktionstemperatur kann von 4 bis 60°C, bevorzugt 30 bis 37°C betragen. Die Substratkonzentration sollte im Bereich von 0,1 bis 5 %, bevorzugt 1 bis 2 % liegen.

Bei höheren oder niedrigeren Temperaturen bzw. pH-Werten als angegeben kann die Reaktion ebenfalls noch durchgeführt werden. Jedoch ist die Moenomycinase dann weniger aktiv.

Als Reaktionsprodukte von Moenomycin A entstehen die im Schema dargestellten Stoffe MB und MC. Das Produkt MA kann durch Inkubation von MB mit MBase bei 30 bis 37°C, bevorzugt bei 35 bis 37°C, und pH 5,5 bis 8,5, bevorzugt pH 6 bis 8, über einen Zeitraum von ca. 24 Stunden erhalten werden.

Die genannten Reaktionsprodukte können als Antibiotikum (beispielsweise MB) bzw. als Synthesebaustein für Transglycosylase Inhibitoren (beispielsweise MA und MC) verwendet werden.

Anhand von Beispielen wird die Erfindung weitergehend beschrieben. Prozentangaben beziehen sich, wenn nichts anderes angegeben, auf das Gewicht.

### Beispiel 1

### Stammhaltung von Bacillus spec. DSM 4675

Bacillus spec. DSM 4675 wird auf dem folgenden festen Nährboden gehalten (Medium 1):

| | |
|---|---|
| Bacto Trypton (Difco) | 10 g/l |
| Hefeextrakt (Difco) | 5 g/l |
| NaCl | 5 g/l |
| Agar | 15 g/l |
| pH 7,2 | |

Das Medium wird auf Reagenzgläser verteilt und 30 Minuten bei 121°C sterilisiert, anschließend abgekühlt, mit der Kultur beimpft und 2-3 Tage bei 37°C inkubiert.

Eine Abschwemmung der gewachsenen Kultur dient als Inokulum der folgenden, Moenomycin-haltigen Hauptkultur (Medium 2):

| | |
|---|---|
| Maisstärke | 40 g/l |
| Sojamehl | 35 g/l |
| Saccharose | 10 g/l |
| CaCO₃ | 8 g/l |
| Cornsteep | 4 g/l |
| CoSO₄ | 20 mg/l |
| ®Genapol (Alkylpolyglykolester) | 5 ml/l |
| Moenomycin A | 3 g/l (sterilfiltriert) |
| pH 7,6 | |

30 ml dieses Mediums pro 300 ml Erlenmeyerkolben werden nach dem Beimpfen für 8-48 Stunden bei 37°C und 190 Upm inkubiert. Die dünnschichtchromatographische Analyse des Kulturfiltrates zeigt, daß als Spaltprodukte des Moenomycin die Verbindungen MA, MB und MC nachweisbar sind und daß das eingesetzte Moenomycin gegen Ende der Reaktion vollständig verschwunden ist.

### Beispiel 2

### Herstellung zellfreier Extrakte

Zur Herstellung zellfreier Extrakte wird Bacillus spec. DSM 4675 im Fermenter angezogen. Dazu werden 10 ml Zell-Abschwemmung von der Agarplatte zum Animpfen einer Vorkultur (500 ml Medium 2 ohne Flavomycin im 2 l Erlenmeyerkolben) verwendet, die anschließend 24 Stunden bei 37°C und 190 Upm inkubiert wird.

Als Hauptkulturstufe dient ein 12 l-Laborfermenter gefüllt mit 9 l Medium 3:

| | |
|---|---|
| Pepton | 12,5 g/l |
| Glycerin | 20,0 g/l |
| Citrat | 2,0 g/l |
| K₂HPO₄ | 1,5 g/l |
| MgSO₄ x 7H₂O | 0,5 g/l |
| FeCl₃ x 6H₂O | 0,04 g/l |
| Desmophen (Propylenglykol) | 5,0 ml/l |
| pH 6,8 | |

Dieser wird mit 500 ml Vorkultur beimpft und 24 Stunden bei 37°C, 300 Upm und einer Belüftungsrate von 0,5 vvm inkubiert.

Die ausgewachsene Kultur wird abzentrifugiert und die Zellpaste in Kaliumphosphatpuffer (pH 7,0) 50 mM (1 g Feuchtzellen + 2 ml Puffer) resuspendiert. Anschließend werden die Zellen mit Ultraschall, French Press® oder Dyno Mill® aufgeschlossen und der resultierende Rohextrakt zur Umsetzung verwendet.

In einem Testansatz mit 100 µl Rohextrakt, 12 mg Moenomycin A und 900 µl Kaliumphosphatpuffer (pH 8,0) 50 mM werden innerhalb von 7-24 Stunden bei 37°C 50 % des eingesetzten Substrates abgebaut. Als Reaktionsprodukte findet man MA, MB und MC.

### Beispiel 3

### Präparative Umsetzung von Moenomycin A

Präparative Umsetzungen werden in einen 12 l-Fermenter mit 8,2 l Kaliumphosphatpuffer (pH 8,0) 50 mM, 800 ml Enzym-Rohextrakt und 100 g Moenomycin A bei 37°C und 100 Upm durchgeführt. Der Verlauf der Umsetzung wird mittels DC verfolgt. Nach 8-48 Stunden wird der gesamte Reaktionsansatz lyophilisiert. Der Moenomycinabbau beträgt im allgemeinen 40-60 % (UV-Analyse aus DC). Als Reaktionsprodukte entstehen MA, MB und MC.

### Beispiel 4

### Herstellung der Abbauprodukte

100 g gefriergetrocknetes Gemisch der enzymatischen Umsetzung wurden in 2 l Wasser aufgenommen und zweimal mit je 2 l Ethylacetat extrahiert. Zur völligen Phasentrennung war hierbei eine Zentrifugation erforderlich. Die vereinigten organischen Extrakte wurde über Natriumsulfat getrocknet, filtriert und zur Trockne eingeengt. Es fielen 0,4 g (0,4 %) eines dunkelbraunen Öls an, welches dünnschichtchromatographisch im System n-Butanol/Essigsäure/Wasser = 3/1/2 an Kieselgel (Merck DC-Alufolien 60 F254), unter Anfärbung mit Molybdatophosphorsäure-Cer-(IV)-sulfat-Sprühreagenz (im folgenden kurz PMS-Reagenz genannt), hauptsächlich aus der Komponente MA (Rf-Wert = 0,85) bestand.

Die verbleibende Wasserphase wurde anschließend zweimal mit je 2 l n-Butanol extrahiert. Auch hierbei war eine Zentrifugation zur Phasentrennung erforderlich. Die vereinigten Butanolphasen wurden anschließend so weit wie möglich eingeengt, der Rückstand wurde in wenig Wasser aufgenommen und abschließend gefriergetrocknet. Es fielen 7,4 g (7,4 %) eines gelblichen Pulvers an, welches in der dünnschichtchromatographischen Untersuchung (mit den oben genannten Bedingungen und gleicher Detektion) hauptsächlich aus der Komponente MB (Rf = 0,52) bestand.

Die somit von den unpolaren Substanzen befreite Wasserphase wurde gefriergetrocknet. Die Menge des dabei anfallenden Rückstandes lag bei 76,9 g (76,9 % bei einer Gesamtmenge von 85 %). Dieses hellgelbe Pulver setzte sich dünnschichtchromatographisch aus einer polaren Hauptsubstanz, MC genannt, (Rf = 0,1), verbliebenem Moenomycin A und Nebenprodukten zusammen.

Die so erhaltenen Rohprodukte der Komponenten MA, MB und MC wurden wie folgt weitergereinigt.

### Beispiel 5

### a) Reinigung des Abbauproduktes MA

400 mg MA-Rohprodukt wurden an 120 g Kieselgel (Merck 60, 15 - 40 mcm) chromatographiert, das auf einen pH-Wert von 7,5 eingestellt war. (Das Säulenmaterial wurde dazu auf folgende Art vorbehandelt: das Kieselgel wurde eine Stunde in 500 ml 2N-HCl gerührt, anschließend abgenutscht und neutral gewaschen. Nun wurde mit 1 N NaOH ein pH-Wert von 7,5 eingestellt und schließlich mit 2 l Wasser und 500 ml Methanol gewaschen. Das so vorbehandelte Material wurde über Nacht bei 120°C getrocknet und aktiviert.) Als Elutionsmittel wurde Chloroform/Ethanol = 1/1 verwendet. Die Substanz wurde in 3 ml Lösungsmittelgemisch auf die Säule gegeben und 216 Fraktionen zu je 2,5 ml aufgefangen. Mit DC-Analytik (DC-Platten und Detektion wie in Beispiel 1, Laufmittelsystem wie Säulenelutionsmittel) wurden die Fraktionen 115-175 vereinigt, an Natriumsulfat getrocknet und schließlich zur Trockne eingedampft. Es fielen 27 mg spektrokopisch reines MA an.

### b) Reinigung des Abbauproduktes MB

3,1 g MB-haltiges Rohprodukt aus der Extraktion wurden an 500 g Kieselgel chromatographiert, welches mit dem unter Beispiel 2 erläuterten Verfahren auf einen pH-Wert von 7,5 eingestellt worden war. Unter Verwendung einer Mitteldruck-Chromatographieanlage (MPLC) wurde mit einem Fluß von 10 ml/min bei einem Druck von 2-5 bar mit Chloroform/Ethanol/Wasser = 4/7/1,5 eluiert. Nach einem Vorlauf von 600 ml wurden 220 Fraktionen zu je 10 ml aufgefangen und nach DC vereinigt. Neben MB-haltigen Mischfraktionen fielen in Fraktion 90-170 nach Eindampfen, Aufnahme in Wasser und Gefriertrocknung 1,28 g reines MB an.

### c) Reinigung des Abbauproduktes MC

2,2 g polares Rohprodukt aus der Wasserphase der Extraktion wurden ebenfalls unter Druck chromatographiert (MPLC). Es wurden 500 g Kieselgel mit einem pH-Wert von 7,5 eingesetzt (Verfahren unter Beispiel 2), als Elutionsmittel wurde Ethylacetat/i-Propanol/Wasser = 4/5/5 verwendet. Nach Suspendieren der Aufgabemenge in Methanol mit 15 g Kieselgel, Abdampfen des Lösungsmittels und Eingabe des so behandelten Trägers in eine Vorsäule, wurde bei einem Fluß von 5 ml/min und einem Druck von 2-4 bar eluiert. Ein Vorlauf von 940 ml ging der anschließenden Fraktionierung in 250 Fraktionen zu je 10 ml voran. Die Fraktionen wurden dünnschichtchromatographisch im System Ethylacetat/i-Propanol/Wasser = 1/1/1 unter Anfärbung mit dem PMS-Reagenz bzw. Detektion der UV-Absorption bei 254 nm geprüft und vereinigt. Neben Mischfraktionen lagen in Fraktion 120-190 nach Einengen zur Wasserphase und anschließender Gefriertrocknung 1,3 g reines MC vor, welches spektroskopisch untersucht wurde.

### Beispiel 6

### Strukturaufklärung der durch Enzym-Abbau aus Moenomycin A erhaltene Produkte M_{A} und M_{B}.

### (Die Ziffern der Strukturen beziehen sich auf das Formelschema auf Seite 19)

Für **M**_{**A**} wurde die Struktur **1a** abgeleitet. Die Strukturzuordnung basiert auf dem ¹³C-NMR-Spektrum von **1a**. Reaktion von **1a** mit Diazomethan ergab den Methylester **1b**, der durch ein ¹H-NMR- und ein EI-Massen-Spektrum charakterisiert ist.
Für **M**_{**B**} wurde anhand des ¹³-C- und des FAB-Massen-Spektrums die Struktur **2** abgeleitet. Hydrierung von **2** ergab das Decahydro-Derivat **3a**, das mit Diazomethan zu **3b** reagierte, das bereits früher auf anderem Wege aus Moenomycin A erhalten worden ist.
Mit den vorgeschlagenen Strukturen stimmt überein, daß **2** (M_{B}) enzymatisch in **1a** (M_{A}) übergeführt werden konnte.

### Beschreibung der Versuche

**1a:**
   ¹³-C-NMR (100,6 MHz, CD₃OD): Moenocinol-Teil: δ = 67,5 (C-1), 123,5 (C-2), 141,6 und 141,8 (C-3 und C-7), 32,3 und 32,5 (C-4 und C-5), 126,7 (C-6), 35,9 (C-8), 40,9 (C-9), 30,7 (C-10), 151,1 (C-11), 33,4 (C-12), 122,7 (C-13), 137,3 (C-14), 36,4 (C-15), 27,7 (C-16), 125,3 (C-17), 132,2 (C-18), 25,9 (C-19), 17,8 (C-20), 16,1 (C-21), 109,2 (C-22), 27,3 (C-23 und C-24), 23,8 (C-25). Glycerinsäure-Teil : δ = 175,9 (C-1), 80,6 (C-2), 64,1 (C-3).
   C₂₈H₄₆O₄ (44,6).
**1b:**
   **1a** wurde in wäßriger Lösung mit ®Dowex-50 (H⁺-Form) in die H⁺-Form überführt. **1a** (H⁺-Form, 18,5 mg, 0,04 mmol) wurde in Methanol (3 ml) gelöst und bei 0°C mit überschüssiger etherischer Diazomethan-Lösung versetzt. Das Reaktionsgemisch wurde 2h bei 0°C und 12h bei 20°C gehalten und dann zur Trockne engedampft. Säulenchromatographie (5 g SiO₂, Petrolether/Essigester 2:1) ergab **1b** (3 mg).
   ¹H-NMR (80 MHz, CDCl₃): δ = 0,96 (s, 6H, CH₃-23 und CH₃-24), 1,61 (s, 6H), 1,68 (s, 3H), 1,73 (s, 3H) (4xCH₃), 1,90-2,12 (Allyl-H's), 2,62 (breites d, J = 7Hz, CH₂-12), 3,78 (s, OCH₃), 3,60-4,30 (OCH₂- und OCH-Signale), 4,62 (breites s, CH₂-22), 4,87-5,47 (olefin. H's).- C₂₉H₄₈O₄ (460.7), MS: m/z (%) = 460 (0,03), 271 (10), 230 (19), 199 (68), 43 (100).
**2:**
   ¹³C-NMR (100.6 MHz, D₂O): Moenocinol-Teil: δ = 68,6 (C-1), 124,5 (C-2), 142,9 (C-3), 34,6 (C-4), 34,0 (C-5, C-10), 128,1 (C-6), 143,6 (C-7), 37,8 (C-8), 44,1 (C-9), 151,4 (C-11), 37,2 (C-12), 123,5 (C-13), 138,3 (C-14), 42,2 (C-15), 29,1 (C-16), 126,9 (C-17), 133,1 (C-18), 28,0 (C-19), 19,9 (C-20), 18,2 (C-21), 111,2 (C-22), 29,7 (C-23, 24), 25,9 (C-25). Glycerinsäure-Teil: δ = 179.0 (C-1), 81,8 (C-2), 68,6 (C-3).- C₂₈H₄₇O₇P (526,7), FAB-MS (Matrix: DMSO/Glycerin): m/z = 615 (M-3H+4Na)⁺, 593 (M-2H+3Na)⁺, 571 (M-H+2Na)⁺, 492, 267, 231, 185, 165, 143, 115.
**3a:**
   **2** (14,9 mg, 28,3 µmol) und PtO₂ (4 mg) wurden in Methanol (3ml) und Essigsäure (50 µl) 3 Tage in einer H₂-Atmosphäre unter Normalbedingungen gerührt. Abfiltrieren vom Katalysator und Eindampfen ergab **3a** (13,5 mg).- C₂₈H₅₇O₇P (536,7), FAB-MS (Matrix: DMSO/Glycerin): m/z = 625 (M-3H+4Na)⁺, 603 (M-2H+3Na)⁺, 581 (M-H+2Na)⁺, 558 (M-H+Na)⁺, 514, 500, 498, 432, 404, 362, 340, 298, 288, 266, 186, 164, 142, 115, 93.
**3b:**
   **3a** wurde in wäßriger Lösung mit dem Ionenaustauscher (Dowex-50, H⁺-Form) behandelt, um alle Säurefunktionen freizusetzen. Nach Abfiltrieren vom Harz wurde lyophilisiert. 8,5 mg (15,9 µmol) der so behandelten Probe wurden in Methanol (2 ml) gelöst. Bei 0°C wurde etherische Diazomethan-Lösung im Überschuß zugesetzt. Der Ansatz blieb 2h bei 0°C und 12h bei 20°C stehen. Eindampfen und Säulenchromatographie (5 g SiO₂, Petrolether/Essigester 1:1) ergab **3b** (4,0 mg).
   ¹H-NMR (80 20 MHz, CDCl₃): δ = 3,75 (s, OCH₃ und 2 d, 3J_{H,P} = 10 und 12 Hz, P(OCH₃)₂), 3,20 - 4,40 (OCH₂- und OCH-Multipletts),-C₁₃H₆₃O₇P (578,8), MS: m/z (%) = 563 (0,1, M-CH₃), 519 (1, M-COOCH₃), 452 (1, M-(CH₃O)₂P(O)OH), 381 (3, M-C₁₄H₂₉, Bruch der Bindung zwischen C-8 und C-9 des 25 Perhydromoenocinol-Teils), 229 (8, **a**), 212 (6, **b**), 127 (20), 57 (100).

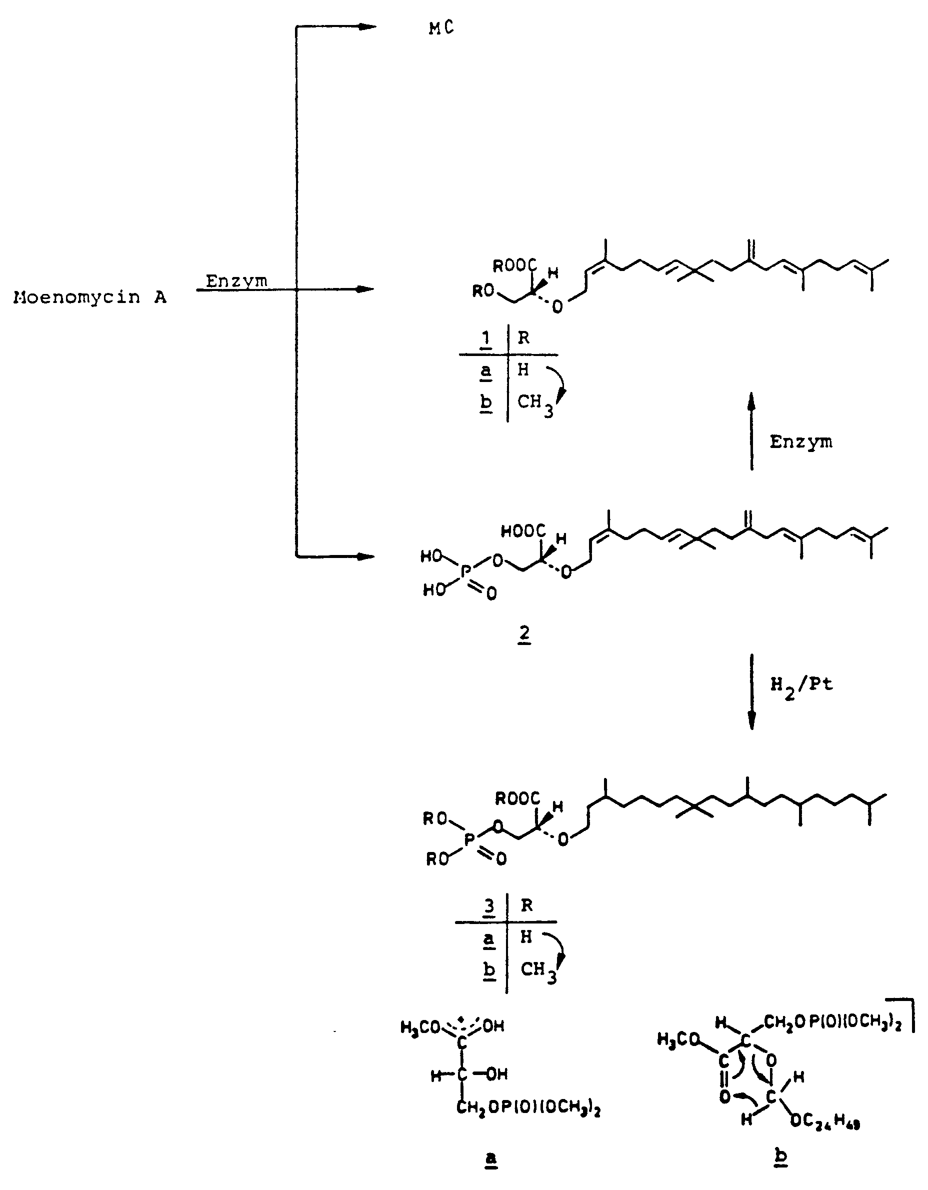

### Beispiel 7

### Antibiotische Aktivitäten der Spaltprodukte

Zur Bestimmung der antibakteriellen Aktivitäten wurde ein Agar-Verdünnungs-Test mit Mueller-Hinton-Agar durchgeführt (Antibiotics in Laboratory Medicine, V. Lorian, Ed., Baltimore 1986, Seiten 1-10).

| Spaltprodukte | Minimale Hemmkonzentration (µg/ml) | | |
|---|---|---|---|
| | Str. pyogenes 77 | Staph. aureus 503 | E. coli DC2 |
| MA | > 100 | > 100 | > 100 |
| MB | 3,125 | 25 | > 100 |
| MC | > 100 | > 100 | > 100 |

### Beispiel 8

### Transglycosylase-Test

Die Hemmung der Polymerisation der Peptidoglycan-Zuckerketten durch die Spaltprodukte wurde gemäß dem von Izaki beschriebenen Test (J. Biol. Chem. 243, 3180-3192, 1968) mit Lipid-Intermediaten der Zellmembran von E. coli K 12 durchgeführt.

Dabei zeigt sich, daß Moenomycin A (20 ug/ml) die Transglycosylase-Reaktion zu 52,7 % und das Spaltprodukt MB das Enzym zu 32,9 % hemmt.

## Patentansprüche (Patentansprüche für folgende(n) Vertragsstaat(en): AT, BE, CH, DE, FR, GB, IT, LI, LU, NL, SE)

1. Bacillus spec. DSM 4675 sowie seine Varianten und Mutanten.

2. Die Verbindung der Formel I

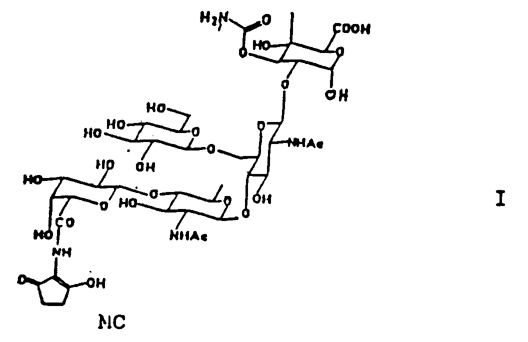

3. Die Verbindung der allgemeinen Formel II, 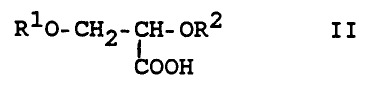 in der R¹ Wasserstoff oder eine Phosphono-Gruppe und R² eine verzweigte oder unverzweigte, gesättigte oder ungesättigte (C₅ bis C₅₅)-Alkylgruppe ist.

4. Die Verbindung nach Anspruch 3, dadurch gekennzeichnet, daß die Alkylgruppe eine Kettenlänge von 10 bis 30 C-Atomen besitzt.

5. Die Verbindung nach Anspruch 3 oder 4, dadurch gekennzeichnet, daß die Alkylgruppe eine Kettenlänge von 20 bis 25 C-Atomen besitzt.

6. Moenomycinase gekennzeichnet durch
- Spaltung der Phosphoglykolipidantibiotika an der phosphoglykosidischen Bindung
- ein pH-Optimum von 8,0 bis 8,5
- ein Temperatur-Optimum von 45 bis 55°C und
- einen Kₘ-Wert von 4 bis 10 mmolar bezogen auf Moenomycin A als Substrat

7. Enzym aus Bacillus spec. DSM 4675, dadurch gekennzeichnet, daß es die Verbindung nach Anspruch 3 dephosphoryliert und über 37°C sowie ab pH 5 mit sinkendem pH inaktiviert wird.

8. Verfahren zur Herstellung der Verbindungen der Formel I nach Anspruch 2, dadurch gekennzeichnet, daß man Moenomycin A mit Bacillus spec. DSM 4675 oder einem daraus gewonnenen Enzymisolat inkubiert.

9. Verfahren zur Herstellung der Verbindung der allgemeinen Formel II nach Anspruch 3, dadurch gekennzeichnet, daß man Phosphoglykolipidantibiotika mit Bacillus spec. DSM 4675 oder einem daraus gewonnenen Enzymisolat inkubiert.

10. Verfahren nach Anspruch 8 oder 9, dadurch gekennzeichnet, daß bei einem pH-Wert von 5,5 bis 8,5 inkubiert wird.

## Patentansprüche (Patentansprüche für folgende(n) Vertragsstaat(en): ES, GR)

1. Bacillus spec. DSM 4675 sowie seine Varianten und Mutanten.

2. Die Verbindung der Formel I

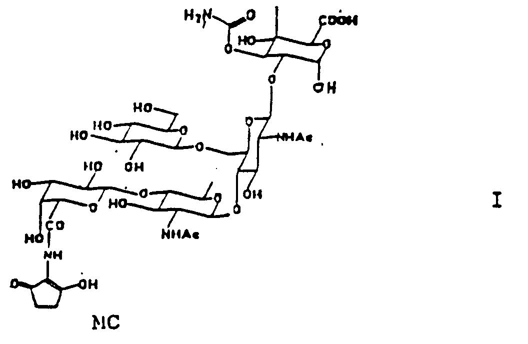

3. Die Verbindung der allgemeinen Formel II, 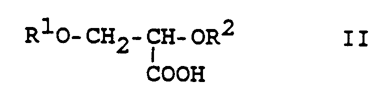 in der R¹ Wasserstoff oder eine Phosphono-Gruppe und R² eine verzweigte oder unverzweigte, gesättigte oder ungesättigte (C₅ bis C₅₅)-Alkylgruppe ist.

4. Die Verbindung nach Anspruch 3, dadurch gekennzeichnet, daß die Alkylgruppe eine Kettenlänge von 10 bis 30 C-Atomen besitzt.

5. Die Verbindung nach Anspruch 3 oder 4, dadurch gekennzeichnet, daß die Alkylgruppe eine Kettenlänge von 20 bis 25 C-Atomen besitzt.

6. Moenomycinase gekennzeichnet durch
- Spaltung der Phosphoglykolipidantibiotika an der phosphoglykosidischen Bindung
- ein pH-Optimum von 8,0 bis 8,5
- ein Temperatur-Optimum von 45 bis 55°C und
- einen Kₘ-Wert von 4 bis 10 umolar bezogen auf Moenomycin A als Substrat

7. Enzym aus Bacillus spec. DSM 4675, dadurch gekennzeichnet, daß es die Verbindung nach Anspruch 3 dephosphoryliert und über 37°C sowie ab pH 5 mit sinkendem pH inaktiviert wird.

8. Verfahren zur Herstellung der Verbindungen der Formel I nach Anspruch 2, dadurch gekennzeichnet, daß man Moenomycin A mit Bacillus spec. DSM 4675 oder einem daraus gewonnenen Enzymisolat inkubiert.

9. Verfahren zur Herstellung der Verbindung der allgemeinen Formel II, 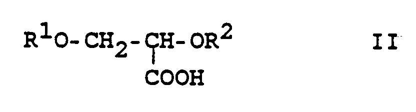 in der R¹ Wasserstoff oder eine Phosphono-Gruppe und R² eine verzweigte oder unverzweigte, gesättigte oder ungesättigte (C₅ bis C₅₅)-Alkylgruppe ist, dadurch gekennzeichnet, daß man Phosphoglykolipidantibiotika mit Bacillus spec. DSM 4675 oder einem daraus gewonnenen Enzymisolat inkubiert.

10. Verfahren nach Anspruch 8 oder 9, dadurch gekennzeichnet, daß bei einem pH-Wert von 5,5 bis 8,5 inkubiert wird.

## Claims (Claims for the following Contracting State(s): AT, BE, CH, DE, FR, GB, IT, LI, LU, NL, SE)

1. Bacillus spec. DSM 4675 and the variants and mutants thereof.

2. The compound of the formula I

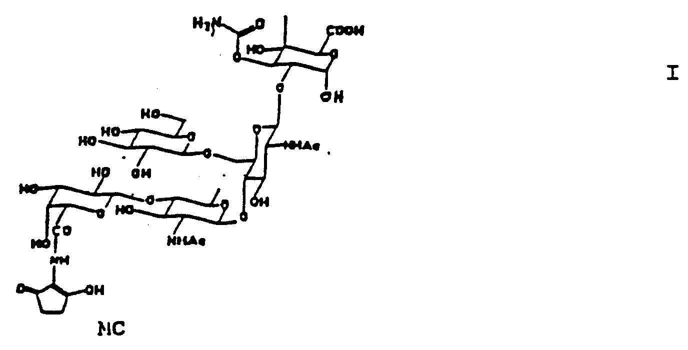

3. The compound of the formula II 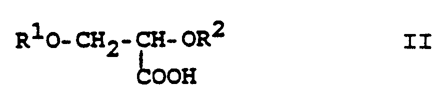 in which R¹ is hydrogen or a phosphono group and R² is a branched or unbranched, saturated or unsaturated (C₅ to C₅₅)-alkyl group.

4. The compound as claimed in claim 3, wherein the alkyl group has a chain length of 10 to 30 carbon atoms.

5. The compound as claimed in claim 3 or 4, wherein the alkyl group has a chain length of 20 to 25 carbon atoms.

6. Moenomycinase having the following characteristics
- cleavage of phosphoglycolipid antibiotics at the phosphoglycosidic linkage
- a pH optimum of 8.0 to 8.5
- a temperature optimum of 45 to 55°C and
- a Kₘ value of 4 to 10 mmolar based on moenomycin A as substrate.

7. An enzyme from Bacillus spec. DSM 4675, which dephosphorylates the compound as claimed in claim 3, and is inactivated above 37°C and with decreasing pH below pH 5.

8. A process for the preparation of compounds of the formula I as claimed in claim 2, which comprises incubating moenomycin A with Bacillus spec. DSM 4675 or an enzyme isolate obtained therefrom.

9. A process for the preparation of the compound of the formula II as claimed in claim 3, which comprises incubating phosphoglycolipid antibiotics with Bacillus spec. DSM 4675 or an enzyme isolate obtained therefrom.

10. The process as claimed in claim 8 or 9, wherein incubation is carried out at a pH of 5.5 to 8.5.

## Claims (Claims for the following Contracting State(s): ES, GR)

1. Bacillus spec. DSM 4675 and the variants and mutants thereof.

2. The compound of the formula I

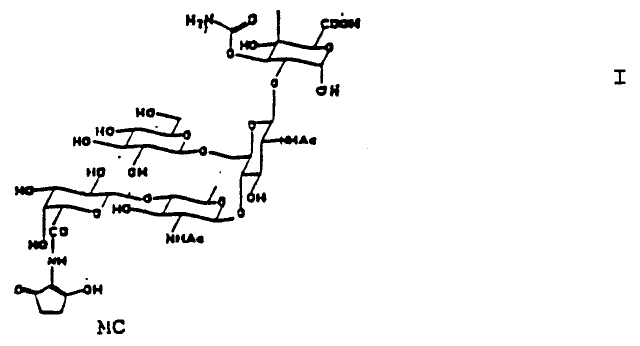

3. The compound of the formula II 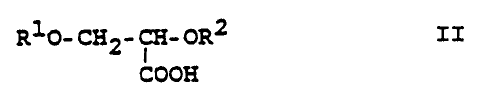 in which R¹ is hydrogen or a phosphono group and R² is a branched or unbranched, saturated or unsaturated (C₅ to C₅₅)-alkyl group.

4. The compound as claimed in claim 3, wherein the alkyl group has a chain length of 10 to 30 carbon atoms.

5. The compound as claimed in claim 3 or 4, wherein the alkyl group has a chain length of 20 to 25 carbon atoms.

6. Moenomycinase having the following characteristics
- cleavage of phosphoglycolipid antibiotics at the phosphoglycosidic linkage
- a pH optimum of 8.0 to 8.5
- a temperature optimum of 45 to 55°C and
- a Kₘ value of 4 to 10 mmolar based on moenomycin A as substrate.

7. An enzyme from Bacillus spec. DSM 4675, which dephosphorylates the compound as claimed in claim 3, and is inactivated above 37°C and with decreasing pH below pH 5.

8. A process for the preparation of compounds of the formula I as claimed in claim 2, which comprises incubating moenomycin A with Bacillus spec. DSM 4675 or an enzyme isolate obtained therefrom.

9. A process for the preparation of the compound of the formula II
in which R¹ is hydrogen or a phosphono group and R² is a branched or unbranched, saturated or unsaturated (C₅ to C₅₅)-alkyl group, which comprises incubating phosphoglycolipid antibiotics with Bacillus spec. DSM 4675 or an enzyme isolate obtained therefrom.

10. The process as claimed in claim 8 or 9, wherein incubation is carried out at a pH of 5.5 to 8.5.

## Revendications (Revendications pour l'(les) Etat(s) contractant(s) suivant(s): AT, BE, CH, DE, FR, GB, IT, LI, LU, NL, SE)

1. Bacillus sp. déposée à la DSM sous le numéro 4675 ainsi que ses variantes et mutants.

2. Composé de formule I

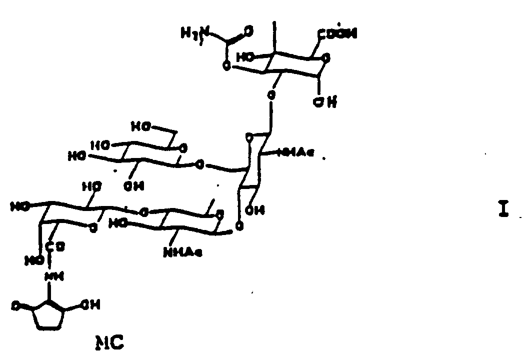

3. Composé de formule générale II 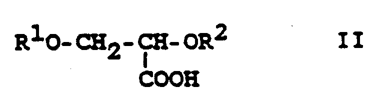 dans laquelle R¹ est hydrogène ou un groupe phosphono et R² est un groupe alkyle en C₅ à c₅₅ linéaire ou ramifié, saturé ou insaturé.

4. Composé selon la revendication 3, caractérisé en ce que le groupe alkyle a une longueur de chaîne de 10 à 30 atomes de carbone.

5. Composé selon la revendication 3 ou 4, caractérisé en ce que le groupe alkyle a une longueur de chaîne de 20 à 25 atomes de carbone.

6. Moenomycinase caractérisée par
- une coupure des antibiotiques de type phosphoglycolipidique au niveau de la liaison phosphoglycosidique;
- un pH optimum de 8,0 à 8,5;
- une température optimale de 45 à 55°C; et
- une valeur de Kₘ comprise entre 4 et 10 mmol calculée sur la base de la moenomycine A en tant que substrat.

7. Enzyme dérivée de Bacillus sp. déposée à la DSM sous le numéro 4675, caractérisée en ce qu'elle réalise la déphosphorylation d'un composé selon la revendication 3 et en ce qu'elle est inactivée au-dessus de 37°C ainsi qu'à partir d'un pH de 5 pour des valeurs décroissantes du pH.

8. Procédé pour la préparation d'un composé de formule I selon la revendication 2, caractérisé en ce que l'on incube la moenomycine A avec Bacillus sp. déposée à la DSM sous le numéro 4675 ou avec un isolat enzymatique obtenu à partir de celui-ci.

9. Procédé pour la préparation d'un composé de formule générale II selon la revendication 3, caractérisé en ce que l'on incube des antibiotiques de type phosphoglycolipidique avec Bacillus sp. déposée à la DSM sous le numéro 4675 ou avec un isolat enzymatique obtenu à partir de celui-ci.

10. Procédé selon la revendication 8 ou 9, caractérisé en ce que l'incubation a lieu à un pH compris entre 5,5 et 8,5.

## Revendications (Revendications pour l'(les) Etat(s) contractant(s) suivant(s): ES, GR)

1. Bacillus sp. déposée à la DSM sous le numéro 4675 ainsi que ses variantes et mutants.

2. Composé de formule I

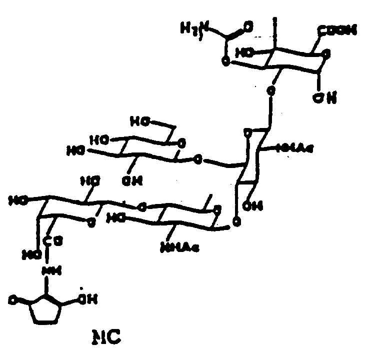

3. Composé de formule générale II 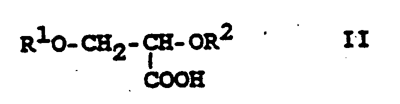 dans laquelle R¹ est hydrogène ou un groupe phosphono et R² est un groupe alkyle en C₅ à c₅₅ linéaire ou ramifié, saturé ou insaturé.

4. Composé selon la revendication 3, caractérisé en ce que le groupe alkyle a une longueur de chaîne de 10 à 30 atomes de carbone.

5. Composé selon la revendication 3 ou 4, caractérisé en ce que le groupe alkyle a une longueur de chaîne de 20 à 25 atomes de carbone.

6. Moenomycinase caractérisée par
- une coupure des antibiotiques de type phosphoglycolipidique au niveau de la liaison phosphoglycosidique;
- un pH optimum de 8,0 à 8,5;
- une température optimale de 45 à 55°C; et
- une valeur de Kₘ comprise entre 4 et 10 mmol calculée sur la base de la moenomycine A en tant que substrat.

7. Enzyme dérivée de Bacillus sp. déposée à la DSM sous le numéro 4675, caractérisée en ce qu'elle réalise la déphosphorylation d'un composé selon la revendication 3 et en ce qu'elle est inactivée au-dessus de 37°C ainsi qu'à partir d'un pH de 5 pour des valeurs décroissantes du pH.

8. Procédé pour la préparation d'un composé de formule I selon la revendication 2, caractérisé en ce que l'on incube la moenomycine A avec Bacillus sp. déposée à la DSM sous le numéro 4675 ou avec un isolat enzymatique obtenu à partir de celui-ci.

9. Procédé pour la préparation d'un composé de formule générale II 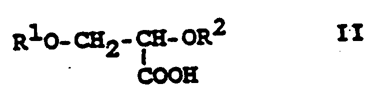 dans laquelle R¹ est hydrogène ou un groupe phoshono et R² est un groupe alkyle en C₅ à C₅₅ linéaire ou ramifié, saturé ou insature, caractérisé en ce que l'on incube des antibiotiques de type phosphoglycolipidique avec Bacillus sp. déposée à la DSM sous le numéro 4675 ou un isolat enzymatique obtenu à partir de celui-ci.

10. Procédé selon la revendication 8 ou 9, caractérisé en ce que l'incubation a lieu à un pH compris entre 5,5 et 8,5.
